(19) Europäisches Patentamt
European Patent Office
Office européen des brevets

(11) Publication number: **0 606 506 A1**

(12) **EUROPEAN PATENT APPLICATION**

(21) Application number: **93100348.7**

(51) Int. Cl.5: **A61K 31/495**, A61K 31/43

(22) Date of filing: **12.01.93**

(43) Date of publication of application:
**20.07.94 Bulletin 94/29**

(84) Designated Contracting States:
**AT BE CH DE DK ES FR GB GR IT LI LU NL SE**

(71) Applicant: **Kim, Yeong Sul**
**Cosmos Mansion n 1002**
**302-62 Ichon-Dong**
**Yongsan-ku Seoul(KR)**

(72) Inventor: **Kim, Yeong Sul**
**Cosmos Mansion n 1002**
**302-62 Ichon-Dong**
**Yongsan-ku Seoul(KR)**

(74) Representative: **KUHNEN, WACKER & PARTNER**
**Alois-Steinecker-Strasse 22**
**D-85354 Freising (DE)**

(54) **Beta-lactamase resistant antibacterial composition.**

(57) The present invention relates to a beta lactamase-resistant antibacterial composition comprising a combination of sulbactam or its salt and one antibacterial compound selected from the compounds I to IV and their salts, as defined in the specification. In the composition of the present invention, since sulbactam inhibits beta-lactamase activity, the compounds I to IV, which are sensitive to beta-lactamase, can act regardless of beta-lactamase and therefore show a superior antibacterial effect in comparison with the single use of the compounds I to IV.

**EP 0 606 506 A1**

BACKGROUND OF THE INVENTION

**Field of the Invention**

The present invention relates to an antibiotic composition showing synergistic activity and more particularly, to a synergistic composition which includes sulbatam and a specific penicillin antibiotic.

Typical antibiotic containing a beta-lactam ring, such as penicillin and cephalosporin antibiotics, are inactivated by beta-lactamases which hydrolyse the beta-lactam ring. Beta-lactamases are secreted by many microorganism species, especially most gram-positive and some gram-negative bacteria. Therefore, disorders caused by such beta-lactamase producing bacteria can be treated only either by using beta-lactamase resistant antibiotics or by using beta-lactamase sensitive antibiotics combined with a drug which can inactivate beta-lactamase.

The present invention relates to a synergistic composition containing sulbactam or a salt thereof and a beta-lactamase sensitive penicillin antibiotic.

The beta lactamase-sensitive penicillin antibiotic which can be used in the present invention is selected from the group consisting of 6-[D(-)-α-amino-p-hydroxyphenylacetamido]penicillanic acid (generic name "amoxicillin" ; hereinafter referred to as "the compound I"), 6-[D-2-(methyleneamino)-2-(4-hydroxyphenyl)-acetamido]penicillanic acid (hereinafter referred to as "the compound II"), 6-[D-2-(4-ethyl-2,3-dioxopiperazinocarbonylamino)-2-(4-hydroxyphenyl)acetamido]penicillanic acid (hereinafter referred to as "the compound III"), 6-[D-2-(D-2-amino-3-N-methylcarbamoyl-propionamido)-2-p-hydroxyphenylacetamido]-penicillanic acid (generic name "aspoxicillin" ; hereinafter referred to as "the compound IV") and their salts.

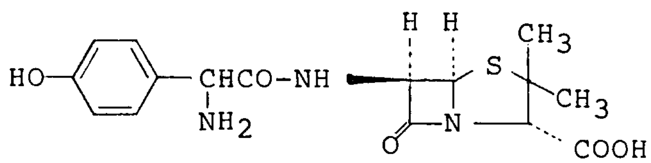
: Compound I

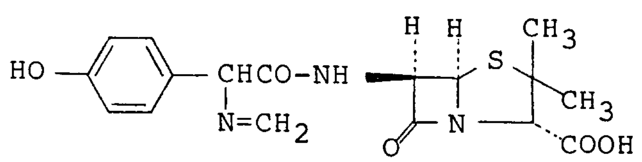
: Compound II

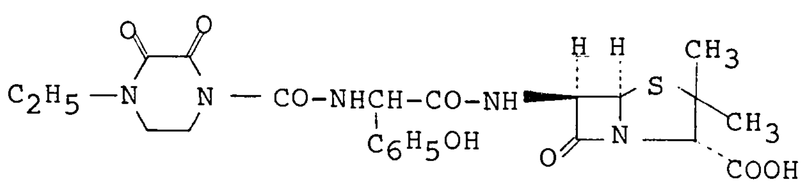
: Compound III

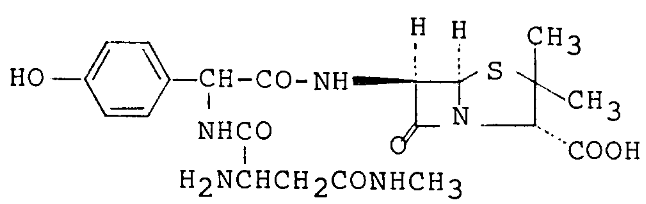
: Compound IV

The term "salt" as used herein for sulbactam and the compounds I through IV means all pharmaceutically acceptable salts conventional in the field of antibiotics. It should be understood that the salts include

all of the alkali or alkaline earth metal salts, inorganic or organic acid addition salts, hydrates, solvates, and the like. The salt most preferred in the present invention is the sodium salt.

Sulbactam as one component of the composition of the present invention is a known beta-lactam antibiotic. However, contrary to conventional beta-lactam antibiotics, sulbactam has a strong affinity to beta-lactamase and thus, after administration, it irreversibly combines with beta-lactamase to inactivate the enzyme [Fu, K.P. and Neu, H.C. Comparative inhibition of beta-lactamase by Novel Beta-lactam compounds, Antimicrob. Agents and Chemother. 15, 171-6 (1979)]. Therefore, sulbactam should not be used alone due to its low antibacterial activity but is generally used in combination with beta-lactam antibiotics sensitive to beta-lactamase in order to prevent the inactivation of beta-lactam antibiotics due to hydrolysis of the beta-lactam ring.

Heretofore, it has been reported that when some penicillin or cephalosporin antibiotics in combination with clavulanic acid or sulbactam are used for anerobic microorganisms such as Bacteroides fragilis, the antibacterial activity of the penicillin or cephalosporin is significantly increased [Greenwood, D. and Eley, A. : In-vitro evaluation of sulbactam, a penicillanic acid sulfone with beta-lactam inhibitory properties, J. Antimicrob. Chemother. 10, 117-123 (1982)]. At present, many studies for such antibiotic combinations have been made.

Up to now, sulbactam, or its salt such as sulbactam sodium, has also been practically used in combination with some beta-lactam antibiotics such as ampicillin or cefoperazone for clinical purposes. However, in the prior art sulbactam has never been used in the form of a combination with the compounds I to IV as in the present invention. Furthermore, since a certain combination between antibiotic compounds cannot be allowed due to their respective antibacterial mechanism and spectrum of treatment, if any antibiotic compound is to be used in combination with sulbactam, a review of the pharmacological properties of the desired combination antibiotic must take place.

According to this, the present inventor has extensively and widely studied the effects of combinations of sulbactam with many beta-lactam antibiotics, including the compounds I to IV which have never been used in combination with sulbactam in the prior art, and thus has identified that the combination of sulbactam and one of compounds I to IV provides synergistic antibiotic activity.

Compounds I and IV, are known beta-lactam antibiotic compounds described in British Patent No. 978,178 and U.S. Patent No. 4,053,609, which are generally named as "amoxicillin" and "aspoxicillin", respectively. Compounds I and IV are antibacterial agents which have been widely used in the antibiotic field but have a disadvantage in that their antibacterial activities are limited because of their decomposition with beta-lactamase.

Compound II is a known penicillin-based antibiotic compound derived from metampicillin (British Patent No. 1,081,093) which also is subject to decomposition by beta-lactamase and hence loss of activity.

Compound III is also a penicillin-based antibiotic compound derived from piperacillin (U.S. Patent No. 4,087,424) which also is subject to decomposition by beta-lactamase and hence loss of activity as is piperacillin.

In the composition of the present invention, since beta-lactamase, which inactivates the compounds I to IV, is inhibited by sulbactam, the combination of sulbactam, or the salts thereof, and any one of the compounds I to IV exhibits a synergistic antibacterial activity. Therefore, when the composition of the present invention is used, a lower amount of the compounds I to IV than their conventional effective antibacterial amounts can provide the desired antibacterial effect.

The compositions of this invention are generally administered to animals, including but not limited to, mammals including humans.

The pharmacologically active composition of this invention can be processed in accordance with conventional methods of galenic pharmacy to produce medicinal agents for administration to patients, e.g., mammals including humans.

The composition of this invention can be employed in admixture with conventional excipients, i.e. pharmaceutically acceptable organic or inorganic carrier substances suitable for parenteral or oral administration which do not deleteriously react with the active components.

For parenteral application, particularly suitable are injectable, sterile solutions, preferably aqueous solutions, as well as suspensions or emulsions. Ampoules are convenient unit dosages.

For oral application, particularly suitable are tablets, liquids, drops, or capsules. Generally, the compounds of this invention are dispensed in unit dosage form in a pharmaceutically acceptable carrier.

In the composition of the present invention the weight ratio of sulbactam to compound I to IV is in the range of 1:0.5 to 1:10, parts by weight, and preferably 1:1 to 1:2, parts by weight.

It will be appreciated that the actual preferred amounts of active compounds in a specific case will vary according to the specific compound, I to IV, being utilized, the particular compositions formulated, the mode

of application, and the particular situs and organism being treated. Dosages for a given host can be determined using conventional considerations, e.g., by customary comparison of the differential activities of the subject compounds and of a known agent, e.g., by means of an appropriate, conventional pharmacological protocol. Thus, the relative amounts of sulbactam to compound I to IV may be varied depending on the effective amount and sensitivity to beta-lactamase of the specific compound I to IV.

The composition of the present invention can be formulated into various conventional pharmaceutical formulations such as injections, capsules, tablets, granules, powders, etc. according to methods conventionally used in the pharmaceutical technical field by using conventional additives, for example, excipients, diluents, binders, stabilizers, preservatives, disintegrators, and the like.

The present invention will be more specifically explained in the following composition examples and experiments.

Composition Examples

**Example 1 (Injections)**

| Component | Content (in 1 vial) |
|---|---|
| Sodium salt of the compound I (amoxicillin sodium)<br>Sodium sulbactam | 250mg (Titer)<br>250mg (Titer) |

Using the above-indicated amounts of sodium salt of the compound I and sodium sulbactam, an injection is prepared according to the conventional method for preparing injections.

**Examples 2 (Injection)**

| Component | Content (in 1 vial) |
|---|---|
| Sodium salt of the compound IV (sodium aspoxicillin)<br>Sodium sulbactam | 400mg (Titer)<br>250mg (Titer) |

Using the above-indicated amounts of sodium salt of the compound IV and sodium sulbactam, an injection is prepared according to the conventional method for preparing injections.

**Example 3 (Capsules)**

| Component | Content (per 1 Cap.) |
|---|---|
| Sodium salt of the compound II<br>Sodium sulbactam<br>Excipients | 500mg (Titer)<br>250mg (Titer)<br>q.s. |

According to the conventional method for preparing capsules, the indicated amounts of the above components are mixed together and then filled in a hard gelatine capsule to prepare the capsule formulation.

**Example 4 (Tablets)**

| Component | Content (per 1 Tab.) |
|---|---|
| Sodium salt of the compound III<br>Sodium sulbactam<br>Formulating aids | 500mg (Titer)<br>250mg (Titer)<br>q.s. |

The indicated amounts of the above components are mixed together to prepare a tablet according to the conventional method for preparing tablets.

**Example 5 (Tablets)**

| Component | Content (per 1 Tab.) |
|---|---|
| Trihydrate of the compound I (amoxicillin trihydrate)<br>Sodium sulbactam<br>Formulating aids | 250mg (Titer)<br>250mg (Titer)<br>q.s. |

The indicated amounts of the above components are mixed together to prepare a tablet according to the conventional method for preparing tablets.

Experiment

To determine the synergistic antibacterial activity of the composition according to the present invention, bacterial strains resistant to the compounds I to IV were selected and then MIC (Minimum Inhibitory Concentration) values of the composition of the present invention against the selected bacterial strains were measured. The obtained MIC value of the composition of the present invention was compared with the MIC value of each of the compounds I, II, III and IV. From the decrease in MIC values the synergistic antibacterial activity of the composition of the present invention can be identified.

A. Experimental method (In vitro actibacterial activity test) :

The antibacterial activity of each of sulbactam and the compounds I, II, III and IV was measured according to the agar dilution method using Mueller Hinton agar. The compounds to be tested were diluted twice in stages and then introduced into the agar plate. On this agar plate the selected bacterial strains were inoculated and then incubated at 37°C for 18 hours. The agar plate was then visually observed to determine the minimum concentration, at which the growth of bacterial strains was not observed, which is then designated as the MIC value of the relevant antibiotic compound.

In the same manner, the MIC value of the compound I, II, III or IV in the presence of sulbactam was measured. In this case, the concentration of sulbactam in each agar plate should be constantly maintained at about 13μg/ml, which corresponds to approximately a quarter of the MIC value of sulbactam, to ensure that the decrease in MIC value of the compounds I - IV in the presence of sulbactam is not affected by the antibacterial activity of sulbactam itself. At this concentration sulbactam does not substantially show its antibacterial activity. As a result of this experiment, the MIC values of each of four kinds of antibiotics, i.e. the compound I, II, III or IV used alone and in the presence of sulbactam were compared to each other and then the synergistic effect of the composition of the present invention was estimated.

The results were described in the following Tables I to IV.

Table I. Antibacterial activity (MIC: $\mu$g/ml) of compound I, sulbactam, and compound I in the presence of sulbactam against bacterial strains resistant to the compound I

| Antibiotics / Strains | MIC value of the compound I | | MIC value of sulbactam |
|---|---|---|---|
| | alone | in the presence of sulbactam(13$\mu$g/ml) | |
| Staphylococcus aureus 285 | 1.563 | 0.195 | 100 |
| Staphylococcus aureus 503 | 1.563 | 0.098 | 100 |
| Escherichia coli O 55 | 1.563 | < 0.002 | 100 |
| Escherichia coli DC 0 | 6.250 | 0.781 | > 100 |
| Escherichia coli DC 2 | 1.563 | 0.781 | 100 |
| Escherichia coli TEM | > 100 | > 100 | > 100 |
| Escherichia coli 1507 E | 3.125 | 0.391 | > 100 |
| Pseudomonas aeruginosa 1771 M | 0.098 | 0.098 | 50 |

Table II. Antibacterial activity (MIC:μg/ml) of compound II, used either alone or in the presence of sulbactam, against bacterial strains resistant to the compound II

| Strains \ Antibiotics | MIC value of the compound II | |
|---|---|---|
| | alone | in the presence of sulbactam(13μg/ml) |
| Staphylococcus aureus 285 | 1.563 | 0.195 |
| Staphylococcus aureus 503 | 1.563 | 0.098 |
| Escherichia coli O 55 | 1.563 | < 0.002 |
| Escherichia coli DC 0 | 6.250 | 3.125 |
| Escherichia coli DC 2 | 1.563 | 0.781 |
| Escherichia coli TEM | > 100 | > 100 |
| Escherichia coli 1507 E | 6.250 | 0.098 |
| Pseudomonas aeruginosa 1771 M | 0.195 | 0.049 |

Table III. Antibacterial activity (MIC:μg/ml) of compound III, used either alone or in the presence of sulbactam, against bacterial strains resistant to the compound III

| Strains \ Antibiotics | MIC value of the compound III | |
|---|---|---|
| | alone | in the presence of sulbactam(13μg/ml) |
| Staphylococcus aureus 285 | 3.125 | 0.781 |
| Staphylococcus aureus 503 | 6.250 | 0.391 |
| Escherichia coli O 55 | 0.195 | < 0.002 |
| Escherichia coli DC 0 | 1.563 | 0.195 |
| Escherichia coli DC 2 | 0.098 | 0.025 |
| Escherichia coli TEM | > 100 | 1.563 |
| Escherichia coli 1507 E | 0.781 | 0.098 |
| Pseudomonas aeruginosa 1771 M | 0.098 | 0.025 |

Table IV. Antibacterial activity (MIC:μg/ml) of compound IV, used either alone or in the presence of sulbactam, against bacterial strains resistant to the compound IV

| Antibiotics / Strains | MIC value of the compound IV | |
|---|---|---|
| | alone | in the presence of sulbactam(13μg/ml) |
| Staphylococcus aureus 285 | 6.250 | 1.563 |
| Staphylococcus aureus 503 | 6.250 | 1.563 |
| Escherichia coli O 55 | 0.781 | < 0.002 |
| Escherichia coli DC 0 | 3.125 | 0.781 |
| Escherichia coli DC 2 | 0.781 | 0.195 |
| Escherichia coli TEM | > 100 | 25 |
| Escherichia coli 1507 E | 3.125 | 0.098 |
| Pseudomonas aeruginosa 1771 M | 0.391 | 0.025 |

B. Results :

From the above experimental results, the synergistic antibacterial activity exhibited by the composition of the present invention is clearly proved.

As can be seen from Tables I and II, when the compound I or the compound II was used in the combination with sulbactam, the MIC value was reduced by at least 2 times to at most 100 times or more in comparision to the single use of the compound I or the compound II.

In addition, as can be seen from Tables III and IV, in comparison to the single use of the compound III or IV the MIC value decreased by 4 to 70 times in the combination of the compound III with sulbactam and by 4 to 100 times in the combination of the compound IV with sulbactam. This result demonstrates that the composition of the present invention comprising any one of the compounds I to IV in combination with sulbactam exhibits a superior synergistic antibacterial activity.

Such synergistic effect could be observed in many strains belonging to Staphylococcus aureus and Escherichia coli. In the case of Pseudomonas aeruginosa strain, it should be specifically noted that although this strain is less resistant to the compound II, III or IV, the MIC value has decreased by approximately 4 to 15 times with the combination of the compound II, III or IV with sulbactam.

From such decrease in the MIC values, it could be demonstrated that since sulbactam contained in the composition of the present invention inhibits the activity of beta-lactamase, the composition according to the present invention can show a strong antibacterial activity even against beta lactamase-producing bacterial strains resistant to the compounds I to IV and further that in the composition of the present invention the compounds I to IV can show thier antibacterial activity even in much lesser amounts than their conventional effective amounts.

Thus, the composition of the present invention, in which sulbactam and any one of the compounds I to IV are combined together, can use even against beta lactamase-producing bacterial strains, for which the compounds I to IV were difficult to use heretofor, and therefore provides an apparent improvement in the field of antibiotics.

## Claims

**1.** A beta lactamase resistant antibacterial composition for treating infectious diseases in the presence of beta-lactamase characterized by a combination of sulbactam or its salt; and one antibacterial compound selected from the group consisting of 6-[D(-)-α-amino-p-hydroxyphenylacetamido]penicillanic acid (the compound I), 6-[D-2-(methyleneamino)-2-(4-hydroxyphenyl)acetamido]penicillanic acid (the compound II), 6-[D-2-(4-ethyl-2,3-dioxopiperazinocarbonylamino)-2-(4-hydroxyphenyl) acetamido]penicillanic acid (the compound III) and 6-[D-2-(D-2-amino-3-N-methylcarbamoylpropionamido)-2-p-hydroxyphenylacetamido]penicillanic acid (the compound IV) and their salts, and which comprises an amount of sulbactam and an amount of one of said antibacterial compounds sufficient to treat said infectious disease in the presence of beta-lactamase together with a pharmaceutically acceptable carrier, adjuvant or excipient therefor.

**2.** The beta-lactamase resistant antibacterial composition for treating infectious diseases of claim 1 which is suitable for injection.

**3.** The beta-lactamase resistant antibacterial composition for treating infectious diseases of claim 1 in the form of a tablet suitable for oral administration.

**4.** The beta-lactamase resistant antibacterial composition for treating infectious diseases of claim 1 in the form of a capsule suitable for oral administration.

**5.** The beta-lactamase resistant antibacterial composition for treating infectious diseases of claim 1, where the ratio of sulbactam to the compounds I to IV is in the range of 1:0.5 to 1:10, parts by weight.

**6.** The beta-lactamase resistant antibacterial composition for treating infectious diseases of claim 5, where the ratio of sulbactam to the compounds I to IV is in the range of 1:1 to 1:2, parts by weight.

**7.** A method of treating a bacterial infection characterized by the presence of beta-lactamase secreting bacteria by administering to one in need of such treatment an amount of a composition comprising sulbactam or its salt and one antibacterial compound selected from the group consisting of 6-[D(-)-$\alpha$ -amino-p-hydroxyphenylacetamido]penicillanic acid (the compound I), 6-[D-2-(methyleneamino)-2-(4-hydroxyphenyl)acetamido] penicillanic acid (the compound II), 6-[D-2-(4-ethyl-2,3-dioxopiperazinocarbonylamino)-2-(4-hydroxyphenyl) acetamido]penicillanic acid (the compound III) and 6-[D-2-(D-2-amino-3-N-methylcarbamoylpropionamido)-2-p-hydroxyphenylacetamido]penicillanic acid (the compound IV) and their salts, sufficient to suppress the growth of the beta-lactamase secreting bacteria.

**8.** The method of claim 7 wherein the composition is orally administered.

**9.** The method of claim 7 wherein the composition is parenterally administered.

European Patent Office

# PARTIAL EUROPEAN SEARCH REPORT

which under Rule 45 of the European Patent Convention shall be considered, for the purposes of subsequent proceedings, as the European search report

Application Number

EP 93 10 0348

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 111, no. 9, 28 August 1989, Columbus, Ohio, US; abstract no. 74648z, SIMONET M. ET AL.. 'Synergy of clavulanic acid sulbactam and tazobactam with amoxycillin against beta-lactamse producing strains of Haemophilus influenzae' page 435 ;column 2 ; * abstract * & J. ANTIMICROB. CHEMOTHER. vol. 23, no. 5, 1989, pages 798 - 800 --- | 1-9 | A 61 K 31/495<br>A 61 K 31/43 |
| X | AM. J. MED. (USA), VOL. 80, NO. 5C, PAGE(S) 21-29, 1986, GUTMANN L. ET AL. 'Synergism and antagonism in double beta-lactam antibiotic combinations' * page 23, column 2, line 11 - line 35 * ----- | 1-9 | |

TECHNICAL FIELDS SEARCHED (Int. Cl.5)

A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet -C-

| Place of search | Date of completion of the search | Examiner |
|---|---|---|
| THE HAGUE | 13-09-1993 | LEHERTE C F M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

European Patent
Office

## CLAIMS INCURRING FEES

The present European patent application comprised at the time of filing more than ten claims.

☐ All claims fees have been paid within the prescribed time limit. The present European search report has been drawn up for all claims.

☐ Only part of the claims fees have been paid within the prescribed time limit. The present European search report has been drawn up for the first ten claims and for those claims for which claims fees have been paid,

namely claims:

☐ No claims fees have been paid within the prescribed time limit. The present European search report has been drawn up for the first ten claims.

## LACK OF UNITY OF INVENTION

The Search Division considers that the present European patent application does not comply with the requirement of unity of invention and relates to several inventions or groups of inventions,
namely:

see sheet -B-

(

☐ All further search fees have been paid within the fixed time limit. The present European search report has been drawn up for all claims.

☐ Only part of the further search fees have been paid within the fixed time limit. The present European search report has been drawn up for those parts of the European patent application which relate to the inventions in respect of which search fees have been paid,

namely claims:

☒ None of the further search fees has been paid within the fixed time limit. The present European search report has been drawn up for those parts of the European patent application which relate to the invention first mentioned in the claims,

namely claims: mentioned in item 1.

European Patent Office

EP 93 10 0348 -B-

## LACK OF UNITY OF INVENTION

The Search Division considers that the present European patent application does not comply with the requirement of unity of invention and relates to several inventions or groups of inventions, namely:

1. Claims 1-9 partially : Combination of sulbactam and amoxicillin.

2. Claims 1-9 partially : Combination of sulbactam and compound II mentioned in the claims.

3. Claims 1-9 partially : Combination of sulbactam and compound III mentioned in the claims.

4. Claims 1-9 partially : Combination of sulbactam and aspoxicillin.

EP 93 100 348 -C-

Remark: Although claims 7-9 are directed to a method of treatment of (diagnostic method practised on) the human/animal body the search has been carried out and based on the alleged effects of the compound/composition.